# EUROPÄISCHE PATENTSCHRIFT

(11) **EP 2 236 082 B1**
(45) Veröffentlichungstag und Bekanntmachung des Hinweises auf die Patenterteilung: **30.11.2011**
(21) Anmeldenummer: 09004930.5
(22) Anmeldetag: 03.04.2009
(51) Int. Cl.: A61B 5/15, A61B 5/151

(54) **Gerät zur Gewinnung und Analyse einer Blutprobe**
Device to obtain and analyse a blood sample
Appareil destiné à la production et à l'analyse d'un échantillon de sang

(43) Veröffentlichungstag der Anmeldung: 06.10.2010
(73) Patentinhaber: Roche Diagnostics GmbH, 68305 Mannheim (DE); F.Hoffmann-La Roche AG, 4070 Basel (CH)
(72) Erfinder: Harttig, Herbert, 67434 Neustadt (DE); Braun, Jürgen, 75365 Calw (DE)
(74) Vertreter: Durm, Frank

(56) Entgegenhaltungen:
- EP-A- 1 402 812
- EP-A- 1 714 613
- EP-A- 1 992 283
- WO-A-2008/083844

## Beschreibung

Die Erfindung betrifft ein Gerät zur Gewinnung und Analyse einer Blutprobe mit den im Oberbegriff des ersten Patentanspruchs angegebenen Merkmalen.

Vor allem Diabetiker sind auf die regelmäßige Selbstkontrolle des Blutzuckerspiegels angewiesen. Dazu muss eine Lanzette mit ihrer Spitze in die Haut eines Körperteils, vorzugsweise in die Fingerkuppe, eingestochen werden. Aus der kleinen Wunde tritt dann ein Blutstropfen aus, der von einem Analysehilfsmittel wie zum Beispiel einem Teststreifen aufgenommen und anschließend einer Analyse zugeführt wird.

Stand der Technik sind kleine automatische Handgeräte mit integrierter Messeinheit und als Einwegartikel (Disposable) ausgebildeter Lanzette. Die jüngste Entwicklung geht hin zu elektrisch angetriebenen Geräten, bei denen nicht nur das Stechen und Aufnehmen einer Blutprobe, sondern auch die anschließende Analyse bis hin zur Ausgabe eines Messergebnisses vollautomatisch abläuft. Dabei ist ein Vorrat von Einweg-Lanzetten Teststreifen in einem Magazin gespeichert, so dass eine Anzahl von Tests durchgeführt werden kann, bevor das Verbrauchsmaterial ausgetauscht werden muss. Der Vorteil eines derart hoch entwickelten Geräts liegt auf der Hand: Musste der Patient früher eine Stechhilfe mit einer ausreichenden Menge von Lanzetten und Teststreifen sowie noch ein Messgerät mitführen und jeweils separat handhaben, so genügt jetzt ein einziges, denkbar einfach zu bedienendes Gerät, um über Tage hinweg für die Kontrolle des Blutzuckerspiegels gerüstet zu sein.

Es ist klar, dass die Konstruktion eines Geräts, das nicht nur die Blutprobe gewinnt, sondern gleich auch automatisch analysiert, eine große Herausforderung für den Konstrukteur darstellt, wenn alle Bedingungen, die an ein solches Gerät heutzutage zu stellen sind, erfüllt werden sollen. Dazu gehören insbesondere einfache und bequeme Bedienung, höchste Zuverlässigkeit, kleine und leichte Bauweise sowie konstruktive Einfachheit, damit die Fertigungskosten in vertretbarem Rahmen für ein Massenprodukt bleiben können. Anders als bei den einfachen Stechhilfen, bei denen der Stechantrieb nur die Lanzette antreiben muss, benötigt ein vollautomatisch arbeitendes Blutzucker-Testgerät einen Antriebsmechanismus, der nicht nur die Lanzette antreibt, sondern auch das Analysehilfsmittel zu dem Körperteil, in das gerade eingestochen wurde, hinführt und dann in einem weiteren Schritt die Blutprobe der Analyse zuführt. Die besondere Problematik dabei ist, dass es wenig Sinn macht, das Analysehilfsmittel in derselben Weise und auf derselben Bahn zu bewegen wie die Lanzette, da das Malysehilfsmittel in Form und Funktion völlig anders ist als die Lanzette. Es muss also eine komplette Bewegungssteuerung vorgesehen werden, die in der Lage ist, das Analyseelement so zu dem fixierten Körperteil zu führen, dass es an einer anderen Stelle in Kontakt mit dem Körperteil kommt als zuvor die Lanzette.

WO 2008/138 455 A1 beschreibt ein Stechsystem mit einem Gerätegehäuse, einer Gehäuseöffnung zum Anlegen eines Körperteils, einer Anzahl von Lanzetten zum Erzeugen einer Stichwunde in dem Körperteil, Probenaufnahmeeinrichtungen zum Aufnehmen einer Körperflüssigkeitsprobe aus der Stichwunde, einem Antrieb, der die Lanzette zum Erzeugen einer Stichwunde und anschließend eine Probenaufnahmeeinrichtung zu der Stichwunde bewegt, und mit einem Kopplungsteil, das eine Lanzette und anschließend eine Probenaufnahmeeinrichtung an den Stechantrieb koppelt, sowie ferner mit einer Bewegungssteuerung, die bewirkt, dass das Kopplungsteil bei einer Probenaufnahmebewegung eine Endposition erreicht, die relativ zu der Endposition, die das Kopplungsteil bei einer Stichbewegung erreicht, seitlich verschoben ist. Lanzetten und Probenaufnahmeeinrichtungen sind auf einem Trägerband angeordnet, das quer durch einen Spalt des Kopplungsteils hindurch transportiert wird. Das Trägerband wird bei der Stichbewegung in Längsrichtung gebogen, so dass sich die Spitze der Lanzette von dem Trägerband abhebt. Bei einer Probenaufnahme wird das Trägerband gleichfalls in seiner Längsrichtung abgeknickt, so dass die Stichwunde flächig von dem umgebogenen Trägerband und der darauf angeordneten Probenaufnahmeeinrichtung, nämlich einem Testfeld, kontaktiert wird. Dabei bewirkt die Bewegungssteuerung eine seitliche Verschiebung quer zur Stichrichtung, so dass die Stichwunde flächig von dem umgebogenen Testfeld kontaktiert wird. Die Bewegungssteuerung umfasst demgemäß eine Kurvensteuerung mit einer ersten Steuerkurve für eine Stichbewegung und einer zweiten Steuerkurve für eine Probenaufnahmebewegung. Diese Steuerkurven verlaufen in einem Führungselement, das sich in Stichrichtung erstreckt. Mittels einer federnden Weiche wird bestimmt, welche der Steuerkurven von einem Steuerkurvenreiter abgefahren wird. Zur Umschaltung zwischen Stichbewegung und Probenaufnahmebewegung muss der Steuerkurvenreiter unter der Weiche hindurch fahren, wobei die Weiche kurzzeitig angehoben wird. Dies erfordert Kraft, die von dem Stechantrieb aufgebracht werden muss und die Leichtgängigkeit und Erschütterungsarmut der Kinematik beeinträchtigt. Nachteilig ist ferner, dass die Weiche ein separat herzustellendes und in die Bewegungssteuerung einzusetzendes Bauteil ist, was zusätzliche Fertigungs- und Montagekosten mit sich bringt.

EP 1 992 283 A1 beschreibt ein Stechsystem mit Lanzetten zum Erzeugen einer Stichwunde und Probeaufnahmeeinrichtungen zum Aufnehmen einer Flüssigkeitsprobe aus der Stichwunde. Zur Ankoppelung der Lanzette für eine Stichbewegung und anschließend einer Probeaufnahmeeinrichtung für eine Probeaufnahmebewegung an einen Antrieb dienen ein Kopplungsteil und eine Bewegungssteuerung, mit der unterschiedliche Bewegungsprofile des Kopplungsteils, nämlich einmal für eine Stichbewegung und zum anderen für eine Probenaufnahmebewegung, erreicht werden können.

Bei einem ersten, in den Figuren 6 und 7 von EP 1 992 283 A1 dargestellten Ausführungsbeispiel umfasst der Antrieb einen Rotor, der auf einer zylindrischen Mantelfläche zwei Nuten mit unterschiedlicher Tiefe aufweist. Diese beiden Nuten bilden jeweils eine Steuerkurve. Das Kopplungsteil hat einen Steuerkurvenreiter, der bei einer Stichbewegung die tiefere Nut abfährt und bei einer Probenaufnahme in die weniger tiefe Nut eingreift. Durch die Drehrichtung des Rotors wird bestimmt, welche der beiden Steuerkurven von dem Steuerkurvenreiter abgefahren wird. Aus derselben Startposition können sowohl eine Probenaufnahmebewegung als auch eine Stichbewegung beginnen.

Bei einem zweiten, in Figur 8 und den folgenden Figuren von EP 1 992 283 A1 dargestellten Ausführungsbeispiel umfasst die Bewegungssteuerung ebenfalls zwei unterschiedliche Steuerkurven, welche aber nicht in der Mantelfläche eines Rotors angeordnet sind, sondern in mindestens einem Führungselement, das sich in Stichrichtung erstreckt. Mittels einer Weiche wird bestimmt, welche der Steuerkurven von dem Steuerkurvenreiter des Kopplungsteils abgefahren werden. Die Führungsbahnen, auf denen der Steuerkurvenreiter gleitet, liegen dabei in derselben Ebene.

Ausgehend von dem beschriebenen Stand der Technik liegt der vorliegenden Erfindung die Aufgabe zugrunde, bei einem Gerät gemäß dem Oberbegriff des ersten Patentanspruchs die Bewegungssteuerung für die Lanzette und das Analysehilfsmittel zu verbessern und zu vereinfachen.

Gelöst wird die Aufgabe gemäß dem kennzeichnenden Teil des ersten Patentanspruchs.

Die Lanzette und das Analysehilfsmittel können sowohl alternativ, das heißt nacheinander, als auch beide gleichzeitig an den Halter angekoppelt werden, zum Beispiel in Form eines integrierten Disposables. In letzterem Fall muss dafür gesorgt werden, dass bei der für die Aufnahme der Blutprobe vorgesehenen Bewegung des Halters nicht auch die Lanzette nochmals in Kontakt mit der Einstichstelle gelangt.

Efindungsgemäß ist die Führungskulisse ein räumliches Gebilde, das sich in alle drei Raumachsen erstreckt. Die Führungsbahnen liegen auf verschiedenen, zueinander parallelen Ebenen, sind also in der Tiefe gestaffelt. Dabei definiert die Führungsbahn der ersten Ebene die Bewegungsbahn des Halters für die Lanzette und die Führungsbahn der zweiten Ebene die Bewegungsbahn des Halters für das Analyseelement. Der gewünschte Querversatz des Analyseelements gegenüber der Stichachse der Lanzette kann dadurch erreicht werden, dass die auf verschiedenen Ebenen liegenden Führungsbahnen unterschiedlich konturiert sind. Die verschiedenen Ebenen der Führungsbahnen verlaufen dabei parallel zur Stichachse und parallel zur Richtung des Querversatzes.

Die Führungskulisse steuert die Bewegung des Halters in einer ersten Achse, die parallel zur Stichachse verläuft, und in einer dazu senkrechten zweiten Achse, die parallel zur Richtung des Querversatzes des Analyseelements verläuft. Stellt man die dreidimensionale Führungskulisse in ein orthogonales Koordinatensystem, so geben die Konturen der Führungsbahnen die Bewegungen des Halters in x- und y-Richtung vor. Die in die Raumtiefe führende Dimension der Führungskulisse (z-Achse) hat keinen Einfluss auf die Bewegungsbahnen der Lanzette und des Analyseelements, sondern dient ausschließlich der Selektion, welche der Führungsbahnen abzufahren ist. Hierzu ist es notwendig, dass das Führungselement unterschiedlich tief in die Führungskulisse eingreift, um stets in Kontakt mit der Führungskulisse zu bleiben.

Die verschiedenen Führungsbahnen, deren Kontur das Führungselement abfahren soll, können einfach dadurch ausgewählt werden, dass von einer auf die andere Ebene übergegangen wird. Allein dadurch, dass das Führungselement bestimmte unterschiedliche Positionen anfährt, wird zwischen den verschiedenen Führungsbahnen hin- und hergewechselt. Dieses Umschalten zwischen den verschiedenen Führungsbahnen gelingt ohne weiteres Bauteil wie zum Beispiel eine Weiche. Die gesamte Bewegungssteuerung ist also auf zwei zusammen wirkende Bauelemente reduziert, nämlich die dreidimensionale Führungskulisse und das darin gleitende Führungselement.

Zur Bewegungssteuerung der Lanzette, die entlang der Stichachse vorschnellt und sofort wieder auf demselben Weg zurückgezogen wird, weist eine der Führungsbahnen im Wesentlichen eine gerade Kontur auf. Diese Kontur bestimmt die Bewegungsbahn des Halters und damit auch der an den Halter angekoppelten Lanzette, wenn diese einen Einstich ausführt.

Der gewünschte Querversatz des Analyseelements gegenüber der Stichachse kann dadurch erzeugt werden, dass die zugehörige Führungsbahn eine gekrümmte Kontur aufweist. Dabei bestimmt der Endpunkt der Krümmung das Maß des Querversatzes und der Radius die Geschwindigkeit, mit der das Analyseelement quer zur Stichachse bewegt wird. In der Praxis bewährt hat sich eine Kontur der Führungsbahn für das Analyseelement, das aus geraden und gebogenen Abschnitten zusammengesetzt ist. Dadurch wird es möglich, das Analyseelement zunächst auf einer gebogenen Bewegungsbahn mit einer Bewegungskomponente senkrecht zur Stichachse zu führen und anschließend, bei Annäherung an die Hautoberfläche, nur noch parallel zur Stichachse.

Damit das Führungselement leicht von einer auf die andere Führungsbahn gelangt, was ja mit einem Wechsel der Ebene einhergeht, umfasst das Führungsprofil vorteilhaft wenigstens eine rampenartig ausgebildete Übergangsbahn, welche die auf verschiedenen Ebenen liegenden Führungsbahnen verbindet. Auf einer solchen Rampe kann das Führungselement von der einen auf die andere Ebene wandern, ohne eine Stufe überwinden zu müssen. Dies schließt natürlich nicht aus, dass das Führungselement auch durch andere Weise die Führungsbahn und damit die Ebene innerhalb der Führungskulisse wechselt, beispielsweise durch "Hinabfallen" oder "Hinaufspringen" einer Stufe. Eine rampenartig ausgebildete Übergangsbahn hat jedoch den Vorteil, dass das Führungselement auf einer stetigen Bahn von Ebene zu Ebene gleiten kann.

Besonders bevorzugt wird eine Ausführung, bei der die Führungskulisse mit dem Halter fest verbunden ist und das Führungselement ortsfest gegenüber dem Gehäuse gelagert ist. In diesem Falle wirkt das Führungselement wie ein Lager, auf dem die Führungskulisse gleitet.

Um es dem Führungselement zu ermöglichen, dass es in Abhängigkeit von seiner Position unterschiedlich tief in die Führungskulisse eingreift, damit es die Konturen der Führungsbahnen abfahren kann, ist das Führungselement vorteilhafterweise als Führungszylinder ausgebildet, der in axialer Richtung verschieblich gelagert ist. Eine in axialer Richtung auf den Führungszylinder wirkende Feder gewährleistet, dass der Führungszylinder auf die Führungsbahnen der Führungskulisse gedrückt und in Eingriff gehalten wird. Ein solches, federnd gelagertes Führungselement gleicht also den Abstand zwischen den Ebenen, auf denen Führungsbahnen angeordnet sind, aus. Alternativ kann das Führungselement teleskopartig ausgebildet sein und beispielsweise aus zwei, vorzugsweise zylindrischen Teilen bestehen, welche gegeneinander in axialer Richtung verschieblich sind.

In zweckmäßiger und vorteilhafter Ausgestaltung hat der Halter für die Lanzette bzw. das Analysehilfsmittel einen Grundkörper in Form eines rechteckigen Quaders, der sich parallel zur Stichachse erstreckt, und ist die Führungskulisse in einer langen Seitenwand des Halters ausgebildet. Beispielsweise kann das Profil der Führungskulisse einstückig in die Seitenwand des Halters eingeformt sein.

Das in die Führungskulisse eingreifende Führungselement erstreckt sich vorzugsweise quer zur Stichachse und damit senkrecht zu den Ebenen der Führungsbahnen.

Da sich der Halter vor allem parallel zur Stichachse hin- und herbewegen soll, ist vorzugsweise an zwei gegenüber liegenden Seitenwänden des Halters je eine Führungskulisse ausgebildet, in die jeweils ein ortsfestes Führungselement eingreift. Die Führungskulissen und Führungselemente sind spiegelbildlich ausgebildet und können einander gegenüberliegen. Der Halter ist dann zwischen den beiden ortsfesten Führungselementen gelagert. Ein seitliches Verkanten des Halters ist damit ausgeschlossen.

Der Halter muss so an den Stechantrieb angekoppelt werden, dass er nicht nur eine Bewegung parallel zur Stichachse, sondern auch den gewünschten Querversatz senkrecht zur Stichachse ausführen kann. Dies kann zum Beispiel dadurch realisiert werden, dass der Halter mit dem Stechantrieb über eine gelenkige Kupplung verbunden ist, die eine Kippbewegung des Halters um eine Achse quer zur Stichachse zulässt. Eine solche gelenkige Kupplung umfasst bevorzugt eine Steckaufnahme für einen Kupplungsstift des Stechantriebs. Die bewegliche Lagerung des Kupplungsstifts in der Steckaufnahme kann durch eine Blattfeder realisiert werden, welche in der Steckaufnahme sitzt und seitlich gegen den Kupplungsstift drückt.

Alternativ können die zwei spiegelbildlichen Führungskulissen und die zugehörigen Führungselemente in Richtung der Stechachse gegeneinander versetzt angeordnet sein. In diesem Fall kann der Halter einfach parallel zur Stichachse nach oben angehoben oder nach unten abgesenkt werden, um den gewünschten Querversatz des Analysehilfsmittels zu bewirken. Ein Kippen des Halters um eine Achse quer zur Stichachse kann so vermieden werden. Natürlich muss der Halter entsprechend leichtgängig gelagert sein. Die Ausgestaltung der Führungsbahnen der Führungskulisse ist entsprechend anzupassen.

Trägt der Halter an seinem in Stichrichtung vorderen Ende eine Klemmvorrichtung, so kann mittels dieser wahlweise und insbesondere abwechselnd eine Einweg-Lanzette oder ein Einweg-Analysehilfsmittel lösbar angeklemmt werden. Nach dem Stich kann die Lanzette gegen ein Analysehilfsmittel ausgetauscht werden. Hat auch das Analysehilfsmittel seinen Dienst getan, kann wieder eine Lanzette angeklemmt werden. Grundsätzlich ist es aber auch möglich, eine Lanzette und ein Analyseelement gleichzeitig anzuklemmen, zum Beispiel bei Verwendung eines integrierten Disposables.

Bevorzugt sind Einweg-Lanzetten und Analysehilfsmittel in Form von Einweg-Teststreifen abwechselnd auf einem Trägerband angeordnet, das schrittweise quer zur Stichrichtung durch die Klemmvorrichtung des Halters gezogen wird. Das Trägerband sorgt auch für den Weitertransport der von dem Teststreifen aufgenommenen Blutprobe zu einer Analyseeinheit, die den Blutzuckergehalt misst.

In vorteilhafter und zweckmäßiger Weiterbildung der Erfindung ist in das Gehäuse eine herausnehmbare Wechselkassette eingesetzt, welche einen Vorrat an Einweg-Lanzetten und Einweg-Teststreifen enthält. Sind die Lanzetten und die Teststreifen abwechselnd auf einem Trägerband angeordnet, so enthält die Wechselkassette zweckmäßigerweise eine Vorratsspule und eine Aufwickelspule. Das Trägerband mit unbenutzten Lanzetten und Teststreifen kann dann von der Vorratsspule abgewickelt und zur Klemmvorrichtung des Halters hin transportiert werden. Nach dem einmaligen Gebrauch werden die Lanzetten bzw. Teststreifen mittels des Trägerbandes wieder von dem Halter wegtransportiert. Das Trägerband mit den verbrauchten Lanzetten und Teststreifen wird dann auf die Aufwickelspule aufgewickelt. Ist der Vorrat an Lanzetten und Teststreifen verbraucht, kann der Benutzer die komplette Wechselkassette einfach und bequem aus dem Gerät herausnehmen und durch eine neue ersetzen.

Besonders bevorzugt wird eine Ausführung, bei welcher der Halter an der Wechselkassette gelagert ist. Grundsätzlich kann der Halter zwar auch unabhängig von der Wechselkassette im Gehäuse beweglich gelagert sein; die Lagerung an oder in der Wechselkassette hat jedoch den Vorteil, dass das unvermeidliche Spiel zwischen Wechselkassette und Gehäuse keinen negativen Einfluss auf die Präzision der Bewegungsabläufe beim An- und Abkoppeln der Lanzetten bzw. Analysehilfsmittel an bzw. von dem Halter hat. Ein weiterer Vorteil besteht darin, dass der Halter zusammen mit der Wechselkassette ausgetauscht wird und somit die Einführung des Trägerbands in die Klemmvorrichtung des Halters mit höchster Zuverlässigkeit bei der Herstellung erfolgen kann.

Bevorzugt ist der Halter zwischen der Vorratsspule und der Aufwickelspule so angeordnet, dass das Trägerband quer zur Stichrichtung durch den Bereich der Klemmvorrichtung des Halters transportiert wird. Dadurch kann der Durchmesser der beiden Spulen gleichzeitig für die Hauptausdehnungsrichtung des Halters genutzt werden, so dass die Wechselkassette insgesamt sehr kompakte Abmessungen hat.

Ein Ausführungsbeispiel der Erfindung wird nachstehend anhand der beigefügten Zeichnungen näher beschrieben. Es zeigen:
- Figur 1: ein komplettes Gerät zur Gewinnung und Analyse einer Blutprobe, in einer perspektivischen Ansicht schräg von oben;
- Figur 2: das Gerät von Fig. 1 von unten, bei abgenommenem Gehäuseunterteil;
- Figur 3: das Gerät von Fig. 1 nach Entfernen des Bodens der Wechselkas- sette;
- Figur 4: das Gerät von Fig. 1 nach vollständiger Herausnahme der Wechselkassette;
- Figur 5: die Anordnung des Halters gemäß Fig. 3, in vergrößertem Maß- stab;
- Figur 6a: den Halter alleine, schräg von unten;
- Figur 6b: die Führungskulisse des Halters, in nochmals vergrößertem Maß- stab;
- Figur 7: einen Vertikalschnitt durch den vorderen Teil des Geräts von Fig. 1, in vergrößertem Maßstab;
- Figur 8: einen Blick in die aufgeschnittene Wechselkassette einer alternati- ven Ausführung des Geräts.

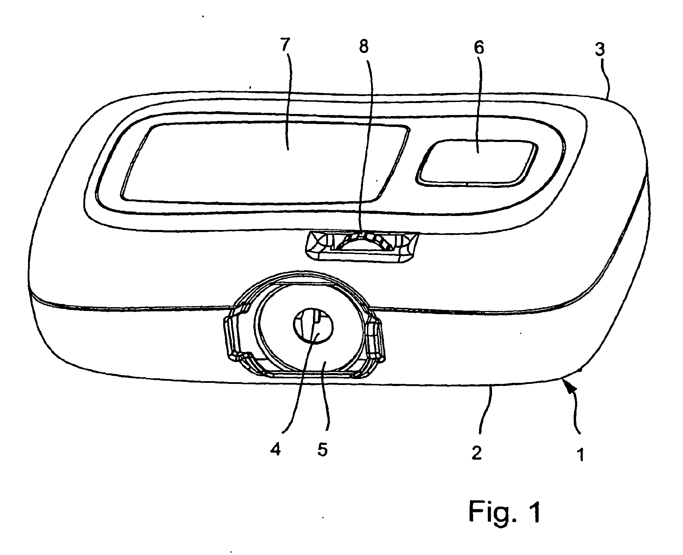

Das in Fig. 1 abgebildete Gerät zur Gewinnung und Analyse einer Blutprobe hat ein flaches rechteckiges Gehäuse 1 mit abgerundeten Ecken. Das Gehäuse 1 ist aus Kunststoff gefertigt und besteht aus einem Gehäuseunterteil 2 und einem Gehäusedeckel 3. An der Vorderseite ist eine runde Öffnung 4 vorgesehen, die von einem Fixierring 5 begrenzt wird. Der Fixierring 5 ist so geformt, dass die Kuppe eines Fingers dagegen gedrückt werden kann. Auf diese Weise lässt sich die Fingerkuppe, aus der eine Blutprobe entnommen werden soll, an dem Gerät fixieren.

Auf dem Gehäusedeckel 3 ist ein Bedienfeld 6 vorgesehen, das Tasten zur Bedienung des Geräts umfasst. Daneben befindet sich eine großflächige Anzeige 7, auf der das Ergebnis des Blutzuckertests abgelesen werden kann. Ein Einstellrad 8 dient der Justierung des Fixierrings 5, um die Einstechtiefe optimal auf die Form des Fingers abzustimmen.

Hinter der Öffnung 4 verbirgt sich eine Stechhilfe mit einer (hier noch nicht sichtbaren) Lanzette 9, die aus der Öffnung 4 herausschnellt, um die Haut des gegen den Fixierring 5 gedrückten Fingers einzustechen und sich anschließend sofort wieder zurückzuziehen. Auf dem gleichen Wege kann anschließend ein Analysehilfsmittel zu dem fixierten Finger hingeführt werden, um einen aus der Stichwunde austretenden Blutstropfen aufzunehmen, der dann einer automatischen Analyse im Inneren des Geräts zugeführt wird.

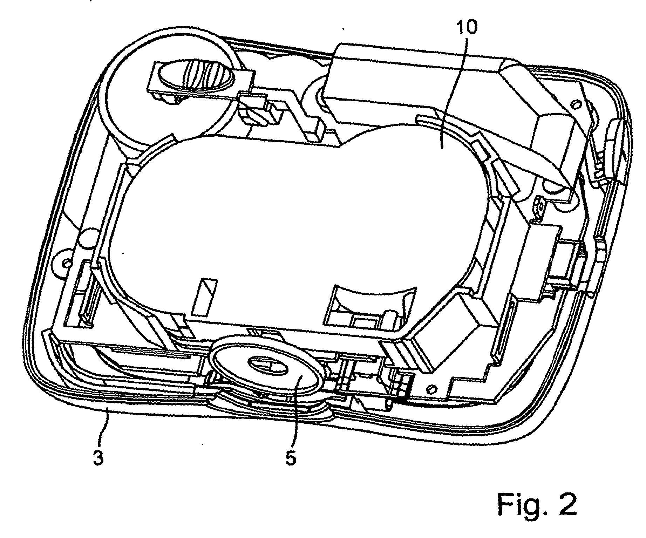

In Fig. 2 ist das Gerät umgedreht und das Gehäuseunterteil 2 entfernt, so dass nur noch der Rand des Gehäusedeckels 3 zu sehen ist. Eine Wechselkassette 10 enthält einen Vorrat von Wegwerf-Lanzetten, die zum einmaligen Gebrauch bestimmt sind. Die Wechselkassette 10 enthält auch einen Vorrat von Analysehilfsmitteln.

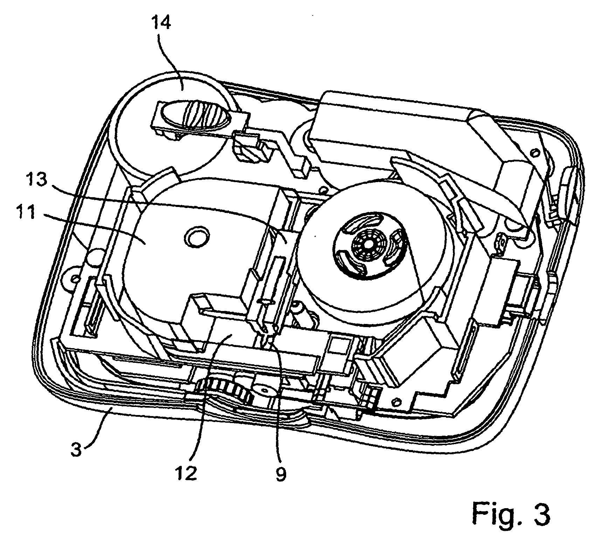

In Fig. 3 wurde der Deckel der Wechselkassette 10 entfernt, so dass der Blick auf ein Vorratsmagazin 11 frei wird. Das Vorratsmagazin 11 enthält ein Trägerband 12, auf dem Einmal-Lanzetten und Analysehilfsmittel abwechselnd hintereinander angeordnet sind. In der Wechselkassette 10 ist neben dem Vorratsmagazin 11 ein Halter 13 angeordnet, an den entweder eine Lanzette oder ein Analysehilfsmittel ankoppelbar ist. Der Halter 13 ist am Boden der Wechselkassette 10 beweglich gelagert.

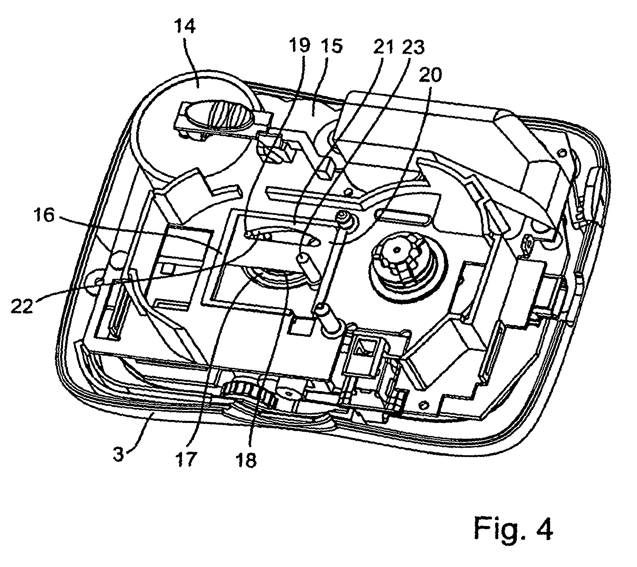

In Fig. 4 ist die Wechselkassette 10 mitsamt dem Halter 13 heraus genommen. Erkennbar ist jetzt der Antriebsmechanismus, der im Wesentlichen aus einem Elektromotor 14, einem Verteilgetriebe 15 und einem Stechantrieb 16 besteht. Der Stechantrieb 16 umfasst einen Rotor 17, der von einer gespannten Spiralfelder 18 in schnelle Drehung versetzt wird. Über einen Mitnehmerstift 19 wird die Drehung des Rotors 17 auf ein Gleitstück 20 übertragen. Der Mitnehmerstift 19 läuft in einer Ausnehmung 21, die kreisabschnittsförmig ausgebildet ist und eine Steuerkurve 22 bildet. Das Gleitstück 20 trägt einen Kupplungsstift 23, der von unten in den Halter 13 (vgl. Fig. 3) eingreift und auf diese Weise die Bewegung des Gleitstücks 20 auf den Halter 13 überträgt.

Das Gleitstück 20 setzt die Drehbewegung des Rotors 17 in eine lineare Bewegung des Kupplungsstifts 23 parallel zur Stichachse um. Nach ungefähr einer halben Umdrehung des Rotors 17 hat der Mitnehmerstift 19 die Steuerkurve 22 abgefahren und seine Endposition in der Ausnehmung 21 erreicht. Um die Spiralfeder 18 wieder zu spannen, muss der Rotor 17 in entgegen gesetzte Richtung gedreht werden. Hierzu ist der Rotor 17 über einen Freilauf an das Verteilgetriebe 15 angekoppelt. Der Stechantrieb wird also mittels des Elektromotors 14 nur gespannt, während der eigentliche Antrieb der Lanzette 9 (vgl. Fig. 1) durch die Kraft der Spiralfelder 18 erfolgt.

Die Ausnehmung 21 kann alternativ auch so ausgeführt sein, dass der Mitnehmerstift 19 eine erste Steuerkurve abfährt, wenn der Rotor 17 durch die gespannte Spiralfeder 18 in eine schnelle Drehung versetzt wird, und eine zweite andere, hier nicht gezeigte Steuerkurve abfährt, wenn der Rotor 17 durch den elektromotorischen Antrieb beim Spannen der Spiralfeder 18 eine Drehung in der Gegenrichtung ausführt. Die zweite Steuerkurve ist dann so ausgeführt, dass die lineare Bewegung des Gleitstücks 20 bei den gegenläufigen Bewegungsrichtungen des Rotors 17 unterschiedliche Strecken umfasst. Dabei ist die Bewegungsbahn beim Spannen der Spiralfeder 18 länger als bei der schnellen Stichbewegung.

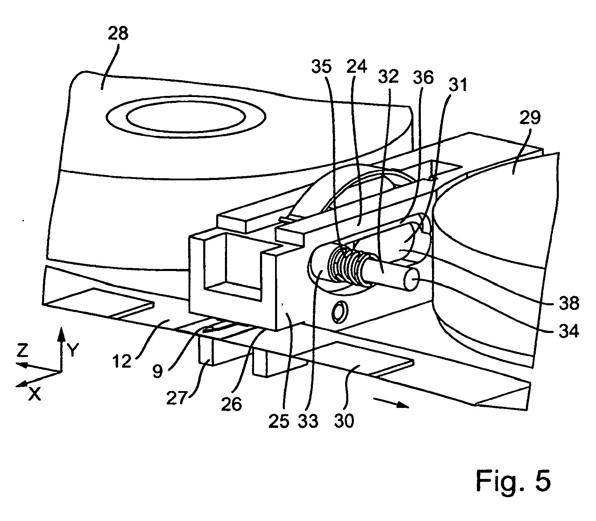

Die Ausschnittsvergrößerung von Fig. 5 zeigt den Bereich um den Halter 13 im Detail.

Der Halter 13 hat einen Grundkörper 24 in Form eines rechteckigen Quaders, der sich parallel zur Stichachse erstreckt. An der vorderen Stirnseite ist ein Greifer 25 angebaut, der einen Schlitz 26 und eine schwenkbewegliche Klemmbacke 27 umfasst. Eine Lanzette 9 befindet sich gerade im Schlitz 26 und wird durch die Klemmbacke 27 am Greifer 25 fixiert. Das Trägerband 12 wird von einer Vorratsspule 28 abgewickelt, quer zur Stichachse durch den Schlitz 26 transportiert und auf einer Wickelspule 29 aufgewickelt. In der Mitte zwischen Vorratsspule 28 und Wickelspule 29 ist der Halter 13 mit Greifer 25 angeordnet.

Vor und hinter der Lanzette 9 sind Analysehilfsmittel in Form von Teststreifen 30 auf dem Trägerband 12 angeordnet. Der Greifer 25 kann also abwechselnd eine Lanzette 9 oder einen Teststreifen 30 ergreifen und an den Halter 13 ankoppeln. Nach dem einmaligen Gebrauch wird die Lanzette 9 bzw. der Teststreifen 30 durch das Trägerband 12 wegtransportiert und am Ende auf der Wickelspule 29 abgelegt. Die Teststreifen 30 durchlaufen vorher noch eine (nicht dargestellte) Analyseeinheit, die den Blutzuckergehalt misst. Vorratsspule 28 und Wickelspule 29 sind im Innern der Wechselkassette 10 angeordnet (vgl. Fig. 2). Ist das Trägerband 12 vollständig durchgelaufen, ist der Vorrat an Lanzetten 9 und Teststreifen 30 erschöpft. Die Wechselkassette 10 kann dann gegen eine neue ausgetauscht werden.

Die Lanzette 9 besteht aus einem Stück Flachstahl mit scharfer Spitze. Die Teststreifen 30 umfassen ein poröses Material, das speziell Blut gut aufsaugt, sowie eine angrenzende Schicht aus Chemikalien, die bei Kontakt mit dem Blut unterschiedlich reagieren, je nachdem, wie hoch der Blutzuckergehalt ist. Diese Reaktion kann zum Beispiel optisch oder elektrisch ausgewertet und das Ergebnis auf der Anzeige 7 (vgl. Fig. 1) dargestellt werden.

Je nachdem, ob eine Lanzette 9 oder ein Teststreifen 30 im Greifer 25 sitzt, muss der Halter 13 unterschiedliche Bewegungen ausführen. Während die Lanzette 9 in einer linearen Bewegung entlang der Stichachse nach vorne geführt und anschließend auf demselben Weg wieder zurückgezogen werden soll, muss ein Teststreifen 30 auf einer alternativen Bewegungsbahn geführt werden, die einen Querversatz senkrecht zur Stichachse der Lanzette 9 umfasst. Dieser Querversatz verläuft hier parallel zur y-Achse des in Fig. 5 eingezeichneten Koordinatensystems.

Die Bewegungssteuerung des Halters 13 erfolgt durch Führungskulissen, die in den langen Seitenwänden des Halters 13 ausgebildet sind. In Fig. 5 ist nur eine (in Stichrichtung rechte) Seitenwand des Halters mit der darin ausgebildeten Führungskulisse 31 zu sehen. Eine zweite, spiegelbildlich ausgebildete Führungskulisse befindet sich in der gegenüber liegenden Seitenwand. Im Folgenden wird nur die eine Führungskulisse 31 weiter beschrieben, wobei klar ist, dass die zweite, spiegelbildliche Führungskulisse in gleicher Weise angeordnet und ausgebildet ist.

In die Führungskulisse 31 greift ein Führungselement 32 ein. Dabei erstreckt sich das Führungselement 32 quer zur Stichachse, also in Richtung der z-Achse des Koordinatensystems. In Abhängigkeit von seiner Position greift das Führungselement 32 unterschiedlich tief in die dreidimensionale Führungskulisse 31 ein.

Das Führungselement 32 wird gebildet durch einen Führungszylinder 33, der auf dem freien Ende eines Lagerzapfens 34 sitzt. Das andere Ende des Lagerzapfens 34 ist in einer (hier nicht dargestellten) Wandung der Wechselkassette 10 axial verschieblich gelagert. Eine in axialer Richtung wirkende Feder 35, die hier als um den Lagerzapfen 34 gewundene Druckfeder ausgeführt ist, drückt den Führungszylinder 33 in die Führungskulisse 31 hinein.

Ein zweites, gleich ausgebildetes Führungselement ist auf der gegenüber liegenden Seite des Halters 13 angeordnet und greift - in der Zeichnung nicht sichtbar - in die zweite Führungskulisse auf der anderen - nicht zu sehenden - Seite des Halters 13 ein.

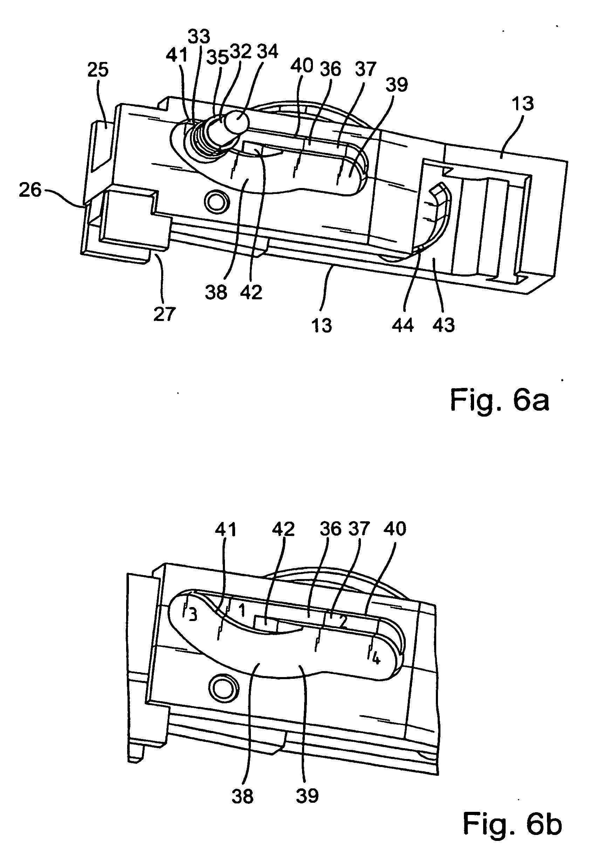

In den Figuren 6a und 6b ist die dreidimensionale Ausbildung der Führungskulisse 31 deutlich zu sehen. Die Führungskulisse 31 umfasst eine erste Führungsbahn 36, deren Boden auf einer ersten Ebene 37 verläuft, und eine zweite Führungsbahn 38, deren Boden auf einer zweiten Ebene 39 verläuft. Dabei sind die Ebenen 37 und 39 zueinander parallel und erstrecken sich sowohl parallel zur Stichachse als auch parallel zur Richtung des Querversatzes, d. h. also, die Ebenen 37 und 39 verlaufen parallel zu der durch die x-Achse und y-Achse aufgespannten Ebene. Ausgehend von der Seitenfläche des Halters 13 liegen die Führungsbahnen 36 und 38 also in unterschiedlicher Tiefe (z-Achse).

Die erste Führungsbahn 36 hat eine im Wesentlichen gerade Kontur 40 und bestimmt damit die lineare Bewegungsbahn der Lanzette 13. Dagegen hat die zweite Führungsbahn 38 eine gekrümmte Kontur 41 und bestimmt die Bewegungsbahn eines Teststreifens 30. Die nach oben verlaufende Kurve der Kontur 41 bewirkt den gewünschten Querversatz des Analyseelements in y-Richtung gegenüber der Stichachse. Zwischen der ersten Führungsbahn 36 und der zweiten Führungsbahn 38 ist eine rampenartig ausgebildete Übergangsbahn 42 vorgesehen. Diese erlaubt es dem Führungszylinder 33, von der zweiten Führungsbahn 38 auf die erste Führungsbahn 36 zu gleiten, obwohl diese auf den verschiedenen Ebenen 37 bzw. 39 liegen. Dabei sorgt die Schraubenfeder 35 dafür, dass der Führungszylinder 33 stets in Eingriff mit einer Führungsbahn 36 bzw. 38 oder der schrägen Übergangsbahn 42 bleibt. Das Führungselement 32 kann also innerhalb der Führungskulisse 31 in x-Richtung hin- und her- und in y-Richtung auf- und abwandern und dabei von der ersten Führungsbahn 36 auf die zweite Führungsbahn 38 wechseln, ohne außer Eingriff zu kommen.

Der Relativbewegung zwischen Halter 13 und Führungselement 32 entspricht die von dem Halter 13 und damit der Lanzette 9 bzw. einem Teststreifen 30 ausgeführte Bewegung in x- und y-Richtung. Die zusätzliche Bewegung des Führungszylinders 33 in z-Richtung hat dagegen keinen Einfluss auf das Bewegungsprofil des Halters 13. Die dritte Dimension der Führungskulisse 31 dient ausschließlich der Umsteuerung von der ersten Führungsbahn 36 auf die zweite Führungsbahn 38, wobei diese Umsteuerung in Abhängigkeit von der Position des Führungselements 32 automatisch erfolgt.

An der Unterseite des Halters 13 ist eine Ausnehmung vorgesehen, die als Steckaufnahme 43 ausgebildet ist. Wenn der Halter 13 in das Gerät eingesetzt ist (vgl. Fig. 3), greift der Kupplungsstift 23 des Gleitstücks 20 von unten in die Steckaufnahme 43 ein. Eine gebogene Blattfeder 44 ragt so in die Steckaufnahme 43 hinein, dass sie von der Seite gegen den Kupplungsstift 23 drückt. Auf diese Weise ist der Halter 13 mit dem Stechantrieb über eine gelenkige Kupplung verbunden, so dass der Halter 13 eine Schwenkbewegung um eine Drehachse quer zur Stichachse (das heißt parallel zur z-Achse in Fig. 5) ausführen kann. Diese Kippbewegung des Halters 13 ermöglicht den Querversatz des vorderen Endes des Halters 13 senkrecht zur Stichachse, wenn in dem Greifer 25 ein Teststreifen 30 sitzt.

In Fig. 6b sind die vier wichtigsten Positionen des Führungselements 32 in der Führungskulisse 31 mit den fortlaufenden Nummern (1), (2), (3) und (4) markiert.

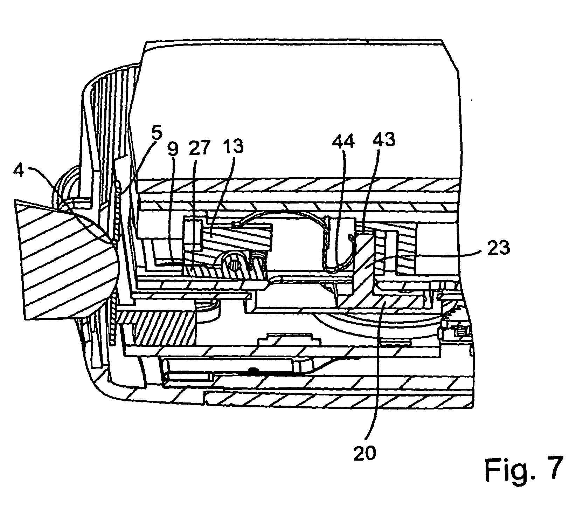

Position (1) ist die Ausgangsposition, bei der sich der Stechantrieb in Ruhestellung befindet, d. h., die Spiralfeder 18 ist gespannt und im Greifer 25 sitzt eine frische Lanzette 9. Nach Auslösen des Stichs durch den Benutzer wird der Halter 13 nach vorne (in Fig. 7 nach links, in Fig. 5 in der x-Richtung) getrieben. Dabei fährt der Führungszylinder 33 die gerade Kontur 40 der ersten Führungsbahn 36 ab, was in eine lineare Bewegung der Lanzette 9 entlang der Stichachse umgesetzt wird.

Die Vorwärtsbewegung der Lanzette wird gestoppt, wenn der Führungszylinder 33 die Umkehrposition (2) erreicht hat. Von da an wird der Halter 13 in umgekehrter Richtung beschleunigt. Das ist die schnelle Rückzugsbewegung der Lanzette 9.

Die Rückzugsbewegung der Lanzette 9 läuft über die Ruheposition (1) hinaus, und der Führungszylinder 33 gelangt in die Position (3). An dieser Stelle verliert der Führungszylinder 33 den Kontakt mit der ersten Führungsbahn 36, die kurz zuvor geendet hat. Unter der Wirkung der Schraubenfeder 35 "fällt" der Führungszylinder 33 nach "unten" auf die tiefer liegende zweite Ebene 39 und gelangt damit auf die zweite Führungsbahn 38. Kurz vor oder kurz nach diesem Zeitpunkt wird die Lanzette 9 aus dem Greifer 25 entfernt und gegen einen unbenutzten Teststreifen 30 ausgetauscht.

Das Gerät ist nun bereit zum Abholen des aus der gerade erzeugten Stichwunde austretenden Blutstropfens. Angetrieben durch den rückwärts drehenden Rotor 17 (vgl. Fig. 4) wird nicht nur der Stechantrieb 16 durch Spannen der Spiralfelder 18 wieder auf den nächsten Stich vorbereitet, sondern auch der Halter 13 erneut in Stichrichtung vorwärts bewegt (nach links in Fig. 7, x-Richtung in Fig. 5). Der Führungszylinder 33 fährt nun die zweite Führungsbahn 38 ab. Deren gekrümmte Kontur 41 bewirkt, dass der Halter 13 nicht nur eine Bewegung parallel zur Stichachse (x-Achse), sondern gleichzeitig auch eine Bewegung senkrecht zur Stichachse (in y-Richtung) ausführt. Entsprechend wird der Teststreifen 30 auf einer anderen Bewegungsbahn als zuvor die Lanzette 9 zur Einstichstelle geführt. Die Ausdehnung der gekrümmten Kontur 41 in y-Richtung bestimmt das Maß des Querversatzes des Teststreifens 30 senkrecht zur Stichachse der Lanzette 9.

Schließlich erreicht der Führungszylinder 33 die Position (4), die einen zweiten Umkehrpunkt darstellt. Wird die Bewegungsrichtung des Halters 13 aus dieser Position heraus erneut umgekehrt, so folgt der Führungszylinder 33 nun dem geraden Abschnitt der Kontur 41 der zweiten Führungsbahn 38 bis zum Anfang der Übergangsbahn 42. Anschließend "klettert" der Führungszylinder 33 über die Rampe der Übergangsbahn 42 nach "oben" zurück auf die erste Führungsbahn 36 und gelangt schließlich zurück an die Ausgangsposition (1).

Die zweite Führungsbahn 38 ist in x-Richtung länger als die erste Führungsbahn 36. Die Bewegungsbahn des Teststreifens 30 war also länger als die Bewegungsbahn der Lanzette 9. Dies ist erforderlich, da der Teststreifen 30 - im Gegensatz zu der starren Lanzette 9 - zusammen mit dem Trägerband rechtwinklig zur Stichachse (nach unten in Fig. 5) abgeknickt werden muss, um flach auf die Stichwunde gedrückt zu werden. Bei der zweiten Bewegung wurde der Halter 13 also ein ganzes Stück weiter in Stichrichtung nach vorne bewegt.

In dem Vertikalschnitt von Fig. 7 liegt die Stichachse genau in der Schnittebene. Es ist deshalb nur die eine Hälfte des Fixierrings 5 zu sehen und die halbe Öffnung 4, durch die ein menschlicher Finger ein kleines Stück hindurchragt. Auch von dem Halter 13 ist nur die dem Betrachter abgewandte Hälfte zu sehen. Die Klemmbacke 27 hält eine Lanzette 9; das Gerät ist also für einen Einstich vorbereitet. Hier gut zu erkennen ist die Ankopplung des Halters 13 an das Gleitstück 20, dessen Kupplungsstift 23 von unten in die Ausnehmung 43 des Halters 13 hineinragt und durch die Blattfeder 44 so festgehalten wird, dass der Halter 13 eine begrenzte Kipp- oder Schwenkbewegung um den Berührpunkt zwischen dem gebogenen Teil der Blattfeder 44 und der in Stichrichtung weisenden Vorderseite des Kupplungsstifts 23 ausführen kann, um den gewünschten Versatz senkrecht zur Stichachse (nach oben in Fig. 7) zu erreichen, wenn anstelle der Lanzette 9 ein Analyseelement angekoppelt ist.

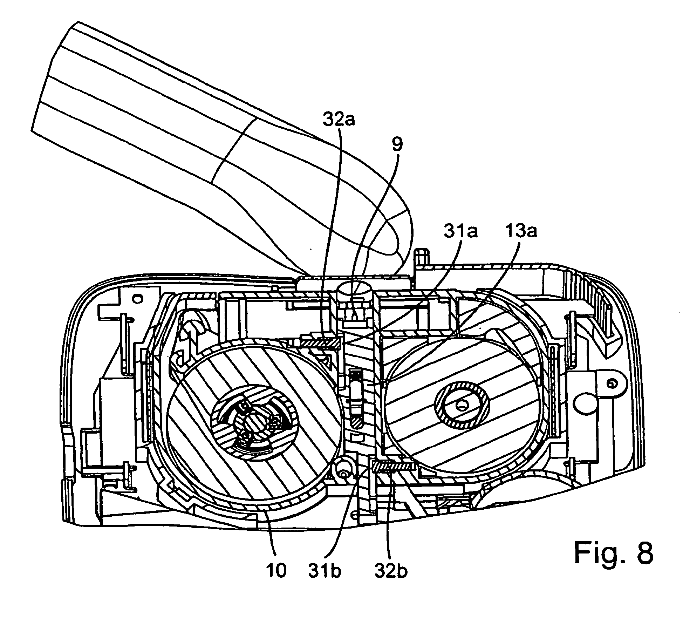

Die in Fig. 8 dargestellte alternative Ausführungsform eines Geräts zur Gewinnung und Analyse einer Blutprobe unterscheidet sich von dem zuvor beschriebenen Gerät dadurch, dass der Halter 13a länger ausgeführt ist, so dass sein hinteres Ende aus der Wechselkassette 10 herausragt. An den zwei langen Seitenwänden des Halters 13a sind wiederum spiegelbildlich ausgeführte Führungskulissen 31 a und 31 b vorgesehen, die hier allerdings nicht einander direkt gegenüberliegen, sondern entlang der Stichachse der Lanzette 9 soweit gegeneinander versetzt angeordnet sind, dass sie im Wesentlichen hintereinander liegen. Entsprechend sind die zugehörigen Führungselemente 32a, 32b um das gleiche Maß in Richtung der Stichachse gegeneinander versetzt angeordnet. Die Anordnung der spiegelbildlichen Führungskulissen 31 a, 31 b und die entsprechende Anordnung der zugehörigen Führungselemente 32a, 32b auf gleicher Höhe, jedoch an unterschiedlichen Längspositionen des Halters 13a hat den Vorteil, dass der Halter 13a über schräge Konturen innerhalb der Führungskulissen 31 a, 31 b als Ganzes angehoben oder abgesenkt werden kann, ohne dass der Halter 13a eine Schwenkbewegung gegenüber dem Kupplungsstift 23 (vergleiche Fig. 7) ausführen muss. Um diese Hubbewegung des Haltes 13a parallel zur Stichachse mit minimalem Kraftaufwand zu realisieren, sollte die Reibung zwischen Kupplungsstift 23 und Steckaufnahme 43 (vgl. Fig. 7) möglichst gering sein.

### Liste der Bezugszeichen

- 1: Gehäuse
- 2: Gehäuseunterteil
- 3: Gehäusedeckel
- 4: Öffnung
- 5: Fixierring
- 6: Bedienfeld
- 7: Anzeige
- 8: Einstellrad
- 9: Lanzette
- 10: Wechselkassette
- 11: Vorratsmagazin
- 12: Trägerband
- 13, 13a: Halter
- 14: Elektromotor
- 15: Verteilgetriebe
- 16: Stechantrieb
- 17: Rotor
- 18: Spiralfeder
- 19: Mitnehmerstift
- 20: Gleitstück
- 21: Ausnehmung
- 22: Steuerkurve
- 23: Kupplungsstift
- 24: Grundkörper (von 13)
- 25: Greifer
- 26: Schlitz
- 27: Klemmbacke
- 28: Vorratsspule
- 29: Aufwickelspule
- 30: Teststreifen
- 31, 31a, 31b: Führungskulissen
- 32, 32a, 32b: Führungselemente
- 33: Führungszylinder
- 34: Lagerzapfen
- 35: Feder
- 36: Erste Führungsbahn
- 37: Erste Ebene
- 38: Zweite Führungsbahn
- 39: Zweite Ebene
- 40: gerade Kontur
- 41: gekrümmte Kontur
- 42: Übergangsbahn
- 43: Steckaufnahme
- 44: Blattfeder

## Patentansprüche

1. Gerät zur Gewinnung und Analyse einer Blutprobe, umfassend
ein Gehäuse (1),
eine am Gehäuse vorgesehene Fixiereinrichtung für ein Körperteil, aus dem eine Blutprobe entnommen werden soll,
wenigstens eine Lanzette (9), die in die Haut des Körperteils eingestochen und wieder zurückgezogen wird,
wenigstens ein Analysehilfsmittel, das zu dem Körperteil hingeführt wird, um einen aus der Stichwunde austretenden Blutstropfen aufzunehmen,
einen beweglich gelagerten Halter (13), an den die Lanzette (9) und das Analysehilfsmittel ankoppelbar sind,
einen Stechantrieb, der den Halter (13) antreibt, um die Bewegungen der Lanzette (9) und des Analysehilfsmittels auszuführen,
eine Bewegungssteuerung, die den Halter (13) auf mindestens zwei alternativen Bewegungsbahnen so führt, dass das Analysehilfsmittel einen Querversatz senkrecht zur Stichachse der Lanzette (9) ausführt,
eine dreidimensionale Führungskulisse (31) mit wenigstens zwei Führungsbahnen (36, 38), wobei die Kontur der ersten Führungsbahn (36) die Bewegungsbahn der Lanzette (9) zum Ausführen eines Einstichs und die Kontur der zweiten Führungsbahn (38) die Bewegungsbahn des Analysehilfsmittels zur Aufnahme der Probenflüssigkeit bestimmt,
wenigstens ein Führungselement (32), das in Abhängigkeit von seiner relativen Position unterschiedlich tief in die Führungskulisse (31) eingreift, um die Konturen (40, 41) der Führungsbahnen (36, 38) abzufahren,
**dadurch gekennzeichnet, dass**
die Führungsbahnen (36, 38) auf verschiedenen, parallel zur Stichachse und zur Richtung des Querversatzes verlaufenden Ebenen (37, 39) liegen und in der Tiefe gestaffelt sind;
das Führungselement (32) zwischen den verschiedenen Führungsbahnen (36, 38) hin- und herwechselt, indem von einer auf die andere Ebene (37, 39) übergegangen wird.

2. Gerät nach Anspruch 1, **dadurch gekennzeichnet, dass** die erste Führungsbahn (36), welche die Bewegungsbahn der Lanzette bestimmt, im Wesentlichen eine gerade Kontur (40) aufweist.

3. Gerät nach Anspruch 1 oder 2, **dadurch gekennzeichnet, dass** die zweite Führungsbahn (38), welche die Bewegungsbahn des Analysehilfsmittels bestimmt, eine gekrümmte Kontur (41) aufweist, wodurch der Querversatz des Analysehilfsmittels gegenüber der Stichachse bewirkt wird.

4. Gerät nach einem der Ansprüche 1 bis 3, **dadurch gekennzeichnet, dass** die Führungskulisse (31) wenigstens eine rampenartig ausgebildete Übergangsbahn (42) umfasst, welche die erste Führungsbahn (36) und die zweite Führungsbahn (38) verbindet.

5. Gerät nach einem der Ansprüche 1 bis 4, **dadurch gekennzeichnet, dass** die Führungskulisse (31) mit dem Halter (13) fest verbunden ist und das Führungselement (32) ortsfest gelagert ist.

6. Gerät nach einem der Ansprüche 1 bis 5, **dadurch gekennzeichnet, dass** das Führungselement (32) durch Federkraft in Eingriff mit der Führungskulisse (31) gehalten wird.

7. Gerät nach Anspruch 6, **dadurch gekennzeichnet, dass** das Führungselement (32) einen Führungszylinder (33) umfasst, der axial verschieblich gelagert ist.

8. Gerät nach Anspruch 7, **dadurch gekennzeichnet, dass** eine in axialer Richtung auf den Führungszylinder (33) wirkende Feder (35) vorgesehen ist.

9. Gerät nach einem der Ansprüche 1 bis 8, **dadurch gekennzeichnet, dass** der Halter (13) einen Grundkörper (24) in Form eines rechteckigen Quaders hat, der sich parallel zur Stichachse erstreckt, und die Führungskulisse (31) in einer langen Seitenwand des Halters (13) ausgebildet ist.

10. Gerät nach einem der Ansprüche 1 bis 9, **dadurch gekennzeichnet, dass** sich das Führungselement (32) quer zur Stichachse erstreckt.

11. Gerät nach Anspruch 9 oder 10, **dadurch gekennzeichnet, dass** an zwei gegenüber liegenden Seitenwänden des Halters (13) zwei spiegelbildlich ausgeführte Führungskulissen (31) vorgesehen sind, in die jeweils ein ortsfestes Führungselement (32) eingreift, so dass der Halter (13) zwischen den beiden Führungselementen gelagert ist.

12. Gerät nach Anspruch 11, **dadurch gekennzeichnet, dass** die Führungskulissen (31) einander gegenüberliegend angeordnet sind.

13. Gerät nach Anspruch 11, **dadurch gekennzeichnet, dass** die beiden Führungskulissen (31a, 31 b) und die zugehörigen Führungselemente (32a, 32b) in Richtung der Stichachse gegeneinander versetzt angeordnet sind.

14. Gerät nach einem der Ansprüche 7 bis 13, **dadurch gekennzeichnet, dass** der Halter (13) mit dem Stechantrieb über eine gelenkige Kupplung verbunden ist, die eine Kippbewegung des Halters (13) um eine Achse senkrecht zur Stichachse zulässt.

15. Gerät nach Anspruch 14, **dadurch gekennzeichnet, dass** der Halter (13) eine Steckaufnahme (43) für einen Kupplungsstift (23) des Stechantriebs aufweist.

16. Gerät nach Anspruch 15, **dadurch gekennzeichnet, dass** in der Steckaufnahme (43) eine Blattfeder (44) sitzt, die von der Seite gegen den Kupplungsstift (23) drückt.

17. Gerät nach einem der Ansprüche 1 bis 16, **dadurch gekennzeichnet, dass** der Halter (13) an seinem in Stichrichtung vorderen Ende eine Klemmvorrichtung trägt, die zum Anklemmen einer Lanzette (9) oder alternativ eines Teststreifens (30) ausgebildet ist.

18. Gerät nach einem der Ansprüche 1 bis 17, **dadurch gekennzeichnet, dass** in das Gehäuse (1) eine herausnehmbare Wechselkassette (10) eingesetzt ist, die einen Vorrat an Lanzetten (9) und Analysehilfsmitteln enthält.

19. Gerät nach Anspruch 18, **dadurch gekennzeichnet, dass** die Wechselkassette (10) eine Vorratsspule (28) mit auf einem Trägerband (12) angeordneten Lanzetten (9) und Teststreifen (30) sowie eine Aufwickelspule (29) für benutzte Lanzetten (9) und Teststreifen (30) enthält.

20. Gerät nach Anspruch 18 oder 19, **dadurch gekennzeichnet, dass** der Halter (13) an der Wechselkassette (10) gelagert ist.

21. Gerät nach Anspruch 20, **dadurch gekennzeichnet, dass** der Halter (13) zwischen der Vorratsspule (28) und der Aufwickelspule (29) so angeordnet ist, dass das Trägerband (12) quer zur Stichrichtung durch den Bereich der Klemmvorrichtung des Halters (13) transportiert wird.

## Claims

1. Device for obtaining and analyzing a blood sample, comprising
a housing (1),
a fixing apparatus, provided on the housing, for a body part from which a blood sample is to be taken,
at least one lancet (9), which is pierced into the skin of the body part and retracted again,
at least one analysis tool, which is guided to the body part, in order to take up a drop of blood exiting from the puncture wound,
a movably mounted holder (13), to which the lancet (9) and the analysis tool can be coupled,
a piercing drive, which drives the holder (13), in order to execute the movements of the lancet (9) and the analysis tool,
a movement controller, which guides the holder (13) on at least two alternative movement paths, the movement of the analysis tool including a transverse offset perpendicular to the puncture axis of the lancet (9),
a three-dimensional control curve (31) having at least two guide paths (36, 38), the contour of the first guide path (36) determining the movement path of the lancet (9) for executing a piercing and the contour of the second guide path (38) determining the movement path of the analysis tool for taking up the sample liquid, and
at least one guide element (32) which, depending on its relative position, engages at different depths in the control curve (31) when travelling along the contours (40, 41) of the guide paths (36, 38),
**characterized in that**
the guide paths (36, 38) lie on different planes (37, 39), which are running parallel to the puncture axis and to the direction of the transverse offset, and are staggered in depth;
the guide element (32) changes back and forth between the various guide paths (36, 38) when passing from one plane to the other.

2. Device according to claim 1, **characterized in that** the first guide path (36), which determines the movement path of the lancet, has an essentially straight contour (40).

3. Device according to claim 1 or 2, **characterized in that** the second guide path (38), which determines the movement path of the analysis tool, has a curved contour (41), the curved contour (41) causing the transverse offset of the analysis tool relative to the puncture axis.

4. Device according to any one of claims 1 to 3, **characterized in that** the control curve (31) comprises at least one ramp-like transition path (42), which connects the first guide path (36) and the second guide path (38).

5. Device according to any one of claims 1 to 4, **characterized in that** the control curve (31) is permanently connected to the holder (13) and the guide element (32) is mounted fixed to the housing (1).

6. Device according to any one of claims 1 to 5, **characterized in that** the guide element (32) is kept in engagement with the control curve (31) by spring force.

7. Device according to claim 6, **characterized in that** the guide element (32) comprises a guide cylinder (33) which is mounted to allow axial displacement thereof.

8. Device according to claim 7, **characterized in that** a spring (35) is provided which acts in the axial direction on the guide cylinder (33).

9. Device according to any one of claims 1 to 8, **characterized in that** the holder (13) has a main body (24) in the form of a rectangular cuboid which extends parallel to the puncture axis, and the control curve (31) is implemented in a long side wall of the holder (13).

10. Device according to any one of claims 1 to 9, **characterized in that** the guide element (32) extends transversely to the puncture axis.

11. Device according to claim 9 or 10, **characterized in that** two mirror-image control curves (31) are provided on two diametrically opposite side walls of the holder (13), each being engaged by a fixed guide element (32), the holder (13) being mounted between the two guide elements.

12. Device according to claim 11, **characterized in that** the control curves (31) are located diametrically opposite to one another.

13. Device according to claim 11, **characterized in that** the two control curves (31a, 31b) and the associated guide elements (32a, 32b) are located offset to one another in the direction of the puncture axis.

14. Device according to any one of claims 7 to 13, **characterized in that** the holder (13) is connected to the piercing drive via an articulated coupling, permitting a tilting movement of the holder (13) around an axis perpendicular to the puncture axis.

15. Device according to claim 14, **characterized in that** the holder (13) has a plug receptacle (43) for a coupling pin (23) of the piercing drive.

16. Device according to claim 15, **characterized in that** a leaf spring (44), which presses from the side against the coupling pin (23), is seated in the plug receptacle (43).

17. Device according to any one of claims 1 to 16, **characterized in that** the holder (13) carries a clamping device on its front end in the puncture direction, which is implemented to clamp on a lancet (9) or alternatively a test strip (30).

18. Device according to any one of claims 1 to 17, **characterized in that** a removable replaceable cassette (10) containing a supply of lancet (9) and analysis tools is inserted into the housing (1).

19. Device according to claim 18, **characterized in that** the replaceable cassette (10) contains a supply coil (28) in which lancets (9) and test strips (30) are located on a carrier band (12), and a winding coil (29) for used lancets (9) and test strips (30).

20. Device according to claim 18 or 19, **characterized in that** the holder (13) is mounted on the replaceable cassette (10).

21. Device according to claim 20, **characterized in that** the holder (13) is located between the supply coil (28) and the winding coil (29) so that the carrier band (12) is transported transversely to the puncture direction through the range of the clamping device of the holder (13).

## Revendications

1. Appareil destiné à l'obtention et à l'analyse d'un échantillon de sang, comprenant
un boîtier (1),
un dispositif de fixation prévu sur le boîtier, pour une partie du corps sur laquelle un échantillon de sang doit être prélevé,
au moins une lancette (9) qui pique la peau et se rétracte ensuite,
au moins un moyen auxiliaire d'analyse qui est transporté vers la partie du corps, afin de recueillir une goutte de sang sortant de la plaie,
un support (13) logé de manière mobile, et auquel la lancette (9) et le moyen auxiliaire d'analyse peuvent être couplés,
une commande de piqûre qui entraîne le support (13), afin d'exécuter les mouvements de la lancette (9) et du moyen auxiliaire d'analyse,
une commande de mouvement qui guide le support (13) sur au moins deux trajectoires alternatives, de sorte que le moyen auxiliaire d'analyse effectue un déplacement transversal perpendiculairement à l'axe de piqûre de la lancette (9),
une coulisse de guidage (31) en trois dimensions dotée d'au moins deux glissières de guidage (36, 38), le contour de la première glissière de guidage (36) déterminant la trajectoire de la lancette (9) pour effectuer une piqûre, et le contour de la deuxième glissière de guidage (38) déterminant la trajectoire du moyen auxiliaire d'analyse pour recueillir le liquide échantillon,
au moins un élément de guidage (32) qui en fonction de sa position relative s'engage dans la coulisse de guidage (31) à une profondeur variable, afin de parcourir les contours (40, 41) des glissières de guidage (36, 38).
**caractérisé en ce que**
les glissières de guidage (36, 38) se trouvent à des niveaux (37, 39) différents, s'étendant parallèlement à l'axe de piqûre et au sens du déplacement transversal, et sont échelonnées en profondeur ;
l'élément de guidage (32) alterne entre les différentes glissières de guidage (36, 38), en passant d'un niveau à l'autre (37, 39).

2. Appareil selon la revendication 1, **caractérisé en ce que** la première glissière de guidage (36) qui détermine la trajectoire de la lancette, présente essentiellement un contour droit (40).

3. Appareil selon la revendication 1 ou 2, **caractérisé en ce que** la deuxième glissière de guidage (38) qui détermine la trajectoire du moyen auxiliaire d'analyse, présente un contour courbe (41), ayant pour effet le déplacement transversal du moyen auxiliaire d'analyse par rapport à l'axe de piqûre.

4. Appareil selon l'une des revendications 1 à 3, **caractérisé en ce que** la coulisse de guidage (31) comprend au moins une glissière de transfert (42) réalisée à la manière d'une rampe, qui relie la première glissière de guidage (36) et la deuxième glissière de guidage (38).

5. Appareil selon l'une des revendications 1 à 4, **caractérisé en ce que** la coulisse de guidage (31) est solidaire du support (13), et l'élément de guidage (32) est logé de manière fixe.

6. Appareil selon l'une des revendications 1 à 5, **caractérisé en ce que** l'élément de guidage (32) est maintenu en prise avec la coulisse de guidage (31) par force élastique.

7. Appareil selon la revendication 6, **caractérisé en ce que** l'élément de guidage (32) comprend un vérin de guidage (33), logé avec possibilité de déplacement axial.

8. Appareil selon la revendication 7, **caractérisé en ce qu'**un ressort (35) est prévu, agissant sur le vérin de guidage (33) dans le sens axial.

9. Appareil selon l'une des revendications 1 à 8, **caractérisé en ce que** le support (13) a un corps de base (24) en forme de parallélépipède rectangle, qui s'étend parallèlement à l'axe de piqûre, et la coulisse de guidage (31) est réalisée dans une paroi latérale longue du support (13).

10. Appareil selon l'une des revendications 1 à 9, **caractérisé en ce que** l'élément de guidage (32) s'étend transversalement à l'axe de piqûre.

11. Appareil selon la revendication 9 ou 10, **caractérisé en ce que** sur deux paroi latérales opposées du support (13) sont prévues deux coulisses de guidage (31) exécutées de manière renversée, dans lesquelles s'engage respectivement un élément de guidage (32) fixe, de sorte que le support (13) est logé entre les deux éléments de guidage.

12. Appareil selon la revendication 11, **caractérisé en ce que** les coulisses de guidage (31) sont disposées en regard l'une de l'autre.

13. Appareil selon la revendication 11, **caractérisé en ce que** les deux coulisses de guidage (31a, 31b) et les éléments de guidage (32a, 32b) associés sont disposés de manière décalée les uns par rapport aux autres dans le sens de l'axe de piqûre.

14. Appareil selon l'une des revendications 7 à 13, **caractérisé en ce que** le support (13) est relié à la commande de piqûre par l'intermédiaire d'un accouplement articulé, qui autorise un mouvement basculant du support (13) autour d'un axe perpendiculaire à l'axe de piqûre.

15. Appareil selon la revendication 14, **caractérisé en ce que** le support (13) comporte un logement d'emboîtement (43) pour une tige d'accouplement (23) de la commande de piqûre.

16. Appareil selon la revendication 15, **caractérisé en ce que** dans le logement d'emboîtement (43) est disposé un ressort à lame (44), qui appuie latéralement contre la tige d'accouplement (23).

17. Appareil selon l'une des revendications 1 à 16, **caractérisé en ce que** le support (13) porte à son extrémité avant dans le sens de la piqûre un dispositif de serrage, réalisé pour fixer une lancette (9) ou alternativement une bandelette de test (30).

18. Appareil selon l'une des revendications 1 à 17, **caractérisé en ce que** dans le boîtier (1) est placée une cassette interchangeable (10) amovible, qui contient une réserve de lancettes (9) et de moyens auxiliaires d'analyse.

19. Appareil selon la revendication 18, **caractérisé en ce que** la cassette interchangeable (10) contient une bobine de réserve (28) avec des lancettes (9) et bandelettes de test (30) disposées sur une bande support (12), ainsi qu'une bobine de réception (29) pour des lancettes (9) et bandelettes de test (30) usagées.

20. Appareil selon la revendication 18 ou 19, **caractérisé en ce que** le support (13) est logé sur la cassette interchangeable (10).

21. Appareil selon la revendication 20, **caractérisé en ce que** le support (13) est disposé entre la bobine de réserve (28) et la bobine de réception (29), de sorte que la bande support (12) est transportée transversalement au sens de la piqûre à travers la zone du dispositif de serrage du support (13).
